# EUROPEAN PATENT SPECIFICATION

(11) **EP 3 130 336 B1**
(45) Date of publication and mention of the grant of the patent: **08.07.2026**
(21) Application number: 16183471.8
(22) Date of filing: 09.08.2016
(51) Int. Cl.: A61K 31/16, A23L 5/00, A23L 29/00

(54) **FOOD AND/OR NUTRACEUTICAL COMPOSITION CONTAINING PALMITOYLETHANOLAMIDE (PEA)**
NAHRUNGSMITTEL UND/ODER NUTRAZEUTISCHE ZUSAMMENSETZUNG MIT PALMITOYLETHANOLAMID (PEA)
ALIMENT ET/OU COMPOSITION NUTRACEUTIQUE CONTENANT DU PALMITOYLÉTHANOLAMIDE (PEA)

(30) Priority: 11.08.2015 IT UB20153066
(43) Date of publication of application: 15.02.2017
(73) Proprietor: Graal S.r.l., 20900 Monza (IT)
(72) Inventor: Seneci, Antonio, 20900 Monza (IT)
(74) Representative: Tagliafico, Giulia

(56) References cited:
- WO-A1-01/24645
- WO-A1-2011/027373
- WO-A2-2005/046580
- DATABASE EMBASE [online] ELSEVIER SCIENCE PUBLISHERS, AMSTERDAM, NL; 20 January 2014 (2014-01-20), KEPPEL HESSELINK J M ET AL: "Palmitoylethanolamide (PEA)-'Promiscuous' anti-inflammatory and analgesic molecule at the interface between nutrition and pharma", XP002757342, Database accession no. EMB-2014086433

## Description

### FIELD OF THE INVENTION

The present invention relates to a food and/or nutraceutical composition comprising palmitoylethanolamide (PEA) in association with a plant extract from *Commiphora myrrha (Nees) Engl.,* beta-caryophyllene and carnosic acid. In particular, it relates to a food and/or nutraceutical composition comprising PEA in association with a plant extract from *Commiphora myrrha (Nees) Engl.* beta-caryophyllene and carnosic acid and an agent that modifies its release in the organism. It also relates to a food and/or nutraceutical composition containing PEA in association with a plant extract from *Commiphora myrrha (Nees) Engl.,* beta-caryophyllene and carnosic acid in various forms of presentation or pharmaceutical forms, namely in buccal, gastro-resistant, multilayered, swallowable, chewable, gastro-filmed or sublingual tablet, or hard capsule filled with liquid and swallowable, chewable, or granulate. Moreover, each of these formulations can be of the conventional type or modified-release (or non-conventional) (for example immediate-release, fast, prolonged or delayed).

### BACKGROUND OF THE INVENTION

PEA was identified over 50 years ago as the active component of fractions having anti-inflammatory activity obtained from the albumen of egg and soy lecithin (Kuehl F.A. et al., (1957), J. Am. Chem. Soc., 79; 5577-5578); in 1965 Bachur and his staff (Bachur N.R. et al. (1965), J. Biol. Chem., 240: 1019-1024) found PEA in various animal tissues, mostly in the brain, the liver and in the skeletal muscle of rats.

Subsequently, PEA has been identified in other animal tissues and cells, such as macrophages and peritoneal leukocytes, rats' plasma, dogs' heart and rats' skin and testicles.

Already in 1993, Professor Rita Levi Montalcini had shown the therapeutic potential of PEA in neurology.

It has now been ascertained that the PEA is produced at tissue level through enzymatic reactions similar to those of anandamide (Di Marzo V. et al., (1994) Nature, 372: 686-691) and can be released with anandamide following depolarization of the neurons (Izzo A.A. et al., (2000), Trends Pharmacol. Sci., 21: 281-282).

In particular, two biosynthetic pathways for PEA have been suggested. The first involves the condensation of ethanolamide with palmitic acid and is catalyzed by a synthetase. Since millimolar concentrations of the two substrates are required so that the enzyme is able to catalyze the reaction (probably because the synthetase normally catalyzes the reverse reaction, namely the hydrolysis of PEA), a second biosynthetic route was proposed. This involves the hydrolysis of a phospholipid precursor, the N-palmitoyl phosphatidylethanolamide (NPPE), catalyzed by a phospholipase. In turn, the NPPE is produced from the coupling of a palmitic radical on the amino group of phosphatidylethanolamine, reaction catalyzed by a calcium-dependent acyltransferase.

PEA has several biological activities similar to anandamide, but unlike the anandamide it does not bind efficiently to cannabinoid receptors CB1 and CB2 (D.M. Lambert, V. Di Marzo, (1999), Curr. Med. Chem., 6: 757- 773).

Therefore, PEA is a substance produced by the human body and acts in the organism as a biological modulator able to re-establish the normal functioning of the tissues. It is defined as "aliamide", or an amide with ALIA effect (Autacoid Local Inflammation/Injury Antagonism). There are situations, in which the capacity of the mast cell to respond to agonistic stimuli is over-expressed and occurs through the release of macromolecules with high pro-inflammatory, algogenic and pruritogenic activity (for example cytokines, vasoactive amines, proteolytic enzymes, bradykinin or histamine). The aliamides exert an antagonistic action of local nature towards the inflammation, modulating the mast cell reactivity.

PEA can also be defined as nutraceutical, which is a food with health supporting properties.

PEA is commercially available as food supplement in various formulations, based on the content percentage of the active ingredient.

Moreover, in some European countries, for example in Italy, Spain, Germany, PEA is commercialized as dietary food for special medical purposes (AFMS), with several trademarks, for example PeaVera^{™} (JP Russell Science), Normast^{®} (Epitech) and Visimast^{®} (Medivis).

It is also interesting to note that PEA has proved to be devoid of side effects, even if used for prolonged periods and/or administered to fragile subjects.

The European patent application EP 2475352 (corresponding to WO 2011/027373) reports a pharmaceutical composition containing an ultra-micronized form of PEA, in which more than 90% by weight of palmitoylethanolamide has particle sizes lower than 6 microns (µm).

The US patent US 5506224 reports a method for treating diseases involving mast cell degranulation, as a consequence of a neurogenic and/or immunogenic hyper-stimulation, comprising the administration of an effective amount of a series of compounds included in a general formula, comprising also the PEA.

Keppel Hesselink J.M. and his group (ELSEVIER Science Publishers, Amsterdam, 20 January 2014) published a review on PEA, reported as a "promiscuous" anti-inflammatory and analgesic molecule at the interface between nutrition and pharma.

WO 01/24645 reports a nutritional or therapeutic composition for oral administration, which comprises a naturally occurring precursor that is metabolized to a compound having anandamide activity for use as a medicament, in which such precursor is a long chain polyunsaturated fatty acid (LCPUFA) (e.g. arachidonic acid ARA or docosahexaenoic acid DHA) or a derivative thereof having a given general formula. According to an embodiment reported in such application, the composition also comprises an inhibitor of an anandamide inactivating enzyme (amidase), which is said to include palmitoylethanolamide. However, no biological effects or further technical results of such hypothetical combination comprising PEA have been shown in such document.

WO 2005/046580 refers to a method of reducing food intake or reducing appetite in a mammal, said method comprising orally administering a fatty acid alkanolamide compound, derivative, homolog, or analog. PEA is reported to be one of such fatty acid alkanolamide compounds.

### DESCRIPTION OF THE INVENTION

It is object of the present invention a food and/or nutraceutical composition comprising PEA in association with with a plant extract from *Commiphora myrrha (Nees) Engl.,* beta-caryophyllene and carnosic acid.

The active ingredients, which, according to the present invention, can be present in association with PEA together with a plant extract from *Commiphora myrrha (Nees) Engl.,* beta-caryophyllene and carnosic acid are reported in Table 1..

Furthermore Table 1 indicates (when applicable) at least one plant extract, in which the corresponding active ingredient is present at significant concentrations. These plant extracts can be used directly in combination with the PEA for the preparation of the composition of the present invention, in place of the corresponding active ingredient in pure or purified form.

**Table 1**

| **PLANT EXTRACT FROM** | **ACTIVE INGREDIENT** |
|---|---|
| *Rosemarinus officinalis L.* | Beta-caryophyllene and carnosic acid |
| *Piper Nigrum L.* | Beta-caryophyllene |
| *Syzygium aromaticum (L.)* Merr. *et L.M. Perry (sin. Caryophyllus aromaticus L.)* or *Eugenia caryophyllata Thunb.* | Eugenol and Beta-caryophyllene |
| *Boswellia serrata roxb.* | Boswellic acids |
| *Boswellia carteri Birdw. (sin. Boswellia sacra Flueck.)* | Boswellic acids |
| *Curcuma longa L. (Curcuma domestica Val., Curcuma domestic Loir., Amomum curcuma Jacq)* | Curcumin and tetrahydrocurcuminoids |
| *Ananas comosus L.* | Bromeline |
| - | L-Theanine |
| *Plectranthus barbatus (syn. Coleus forskohlii)* | Forskolin |
| *Echinacea angustifolia DC.* | Alkylamides and echinacoside |
| *Salvia officinalis L.* | Rosmarinic-acid, carnosol and carnosic acid |
| *Salvia divinorum* Epling & Játiva | Salvinorin-A |
| - | Coenzyme Q10 |
| *Ruta graveolens* | Rutamarin |
| - | Quercetin |
| - | (SAMe) S-Adenosyl-L-methionine |
| *Daucus carota L.* | SOD (superoxide dismutase) |
| *Cucumis melo L.* | SOD (superoxide dismutase) |
| - | L-Glutathione |
| - | NADH (nicotinamide adenine of reduced nucleotide) |
| *Olea europea L.* | Hydroxytyrosol, tyrosol, oleuropein and polyphenols |
| - | Homotaurine/Tramiprosate |
| - | Policosanols |
| - | Trans-resveratrol |
| - | Squalene |
| - | Phosphoserine |
| - | Phosphatidylserine |
| - | Phosphatidylcholine |
| - | Astaxanthin |
| - | Lycopene |
| - | Inositol |
| - | Flavonoids |
| *Polygonum cuspidatum* SIEBOLD & ZUCC. | Resveratrol |
| *Haematococcus pluvialis* FLOTOW | Astaxanthin |
| *Camelia sinensis L.* Kuntze | Epigallocatechin gallate and GABA (gamma-aminobutyric acid) |
| - | Choline |
| - | PABA (Para-aminobenzoic acid) |
| *Garcinia mangostano L.* | Xanthones, alfa- and gamma-mangosteen |
| *Filipendula ulmaria MAX.* | Salicin |
| *Salix alba L.* | Salicin |
| *Gingko biloba L.* | |
| - | Citicoline |
| - | Melatonin |
| *Griffonia simplicifolia BAILL.* | 5-HTP (5-hydroxytryptophan) |
| - | GPC (glycerophosphorylcholine) |
| - | GPE (glycerophosphoethanolamide) |
| *Crataegus monogyna Jacq.* | |
| *Perilla frutescens (L.)* BRITTON. | |
| - | Galactose |
| - | D-mannose |
| - | Lutein |
| - | Zeaxanthin |
| *Tagetes erecta L.* | Lutein and Zeaxanthin |
| *Crocus sativus L.* | |
| *Vaccinium myrtillus L. (or cranberry)* | Proanthocyanidins |
| *Oxycoccus palustris PERS.* | |
| *Serenoa repens (BATRAM) SMALL.* (syn. *Serenoa serrulata HOOK F.)* | |
| *Ajuga reptans L.* | Teupolioside (Teoside^{™}) |
| *Cucurbita pepo L.* | Beta-sitosterol |
| *Prunus africana (Hook. f.) kalkman (syn. Pygeum africanum Hook. f.)* | |
| *Brassica oleracea L.* | Sulforaphane |
| *Harpaghopytum procumbens DC.* | |
| *Uncaria tomentosa WILLD.* | |
| *Zingiber officinalis ROSCH.* | |
| - | Glucosamine |
| - | Chondroitin sulphate |
| - | Hyaluronic acid |
| - | HMB (Hydroxymethylbutyrate) |
| - | Agmatine |
| - | Loperamide |
| *Polypodium leucotomos L.* | Phenolic acids |
| *Punica grantum L.* | Ellagic acid |
| *Theobroma cacao L.* | Epicatechins, catechins and polyphenols |
| *Commiphora myrrha (Nees) Engl.* | |
| *Commiphora mukul Engl.* | |
| *Linum usitatissimum L.* | |
| *Buglossoides arvensis (L.)* I.M. Johnst | |
| *Vitis vinifera L.* (fruit, leaf, seed) | Proanthocyanidins |
| *Sophora japonica L. (Styphnolobium japonicum (L.) Schott)* | Troxerutin |
| *Galium aparine L.* | Asperuloside and iridoid glycosides |
| *Scutellaria baicalensis Georgi* | Baicalin |
| *Apium graveolens L.* | |
| *Fraxinus excelsior L.* | Cumarin |
| *Oenothera biennis L.* | |
| *Ribes nigrum L.* | |
| *Borago officinalis L.* | |
| *Glycine max L.* | Ginesteina |
| *Trifolium pratense L.* | |
| - | N-acetylcistein |
| - | acetyl L-carnitine |
| - | *Serratiopeptidasi* |

Among the associations, the preferred according to the present invention is the following:
the association of PEA (for example, 600 mg per tablet) with carnosic acid (for example 20 mg per tablet), myrrh (for example 50 mg per tablet) and beta-caryophyllene (for example 10 mg per tablet) having an analgesic/anti-pain function in the neurological field (for example useful in the treatment of neuropathic or oncological pain).

The food and/or nutraceutical composition comprising PEA together with a plant extract from *Commiphora myrrha (Nees) Engl.* beta-caryophyllene and carnosic acid, of the present invention optionally further contain other active ingredients not listed in Table 1, as for example other long chain fatty acid amides, lactic acid bacteria, vitamins, trace elements, mineral salts and/or simple or branched amino acids.

Examples of such additional and optional active ingredients are the following: N-oleylethanolamide, N-linoleylethanolammide, *Saccharomyces boulardii, Saccharomyces cerevisiae,* magnesium, potassium, calcium, selenium, zinc, vitamin B3, vitamin B6, vitamin C and vitamin E.

The food and/or nutraceutical composition comprising PEA of the present invention is formulated in various forms of presentation or pharmaceutical forms, namely in solid pharmaceutical formulations (for example tablets, pills, hard capsules filled with liquids or solids, soft capsules, powders, granules or suppositories), semisolid (e.g., gels, ointments, lubricants, creams or pastes) or liquid (e.g. syrups, vials, drops or eye drops). Furthermore, each of these forms can be of the conventional type or modified release (for example immediate-release, fast, prolonged or delayed).

With the expression "conventional pharmaceutical forms" it is meant here, those pharmaceutical forms, for which the release profile (and therefore absorption profile) of the active ingredient depends exclusively on the physical and chemical characteristics of that ingredient, and not by the technological characteristics of the formulation.

The expression "modified-release pharmaceutical forms (or non-conventional)" means here those pharmaceutical forms, for which the release profile (and therefore absorption profile) of the active ingredient depends both on the chemical and physical characteristics of such ingredient that the technological characteristics of the formulation. The factor determining the absorption profile is the release rate of the active ingredient from the pharmaceutical form.

With the expression "agent that modifies the release" of an active ingredient in the organism it is intended here a compound able to modify the rate or the site of dissolution and, consequently, the absorption of an active ingredient by the tissues and the body fluids, with which it comes in contact after administration.

Examples of various classes of such agents are: disintegrating agents (compounds that facilitate the disintegration of solid pharmaceutical forms, increasing the contact surface with the biological fluids and thus increasing the rate of release and absorption of the active ingredient); surfactants (compounds, which are added to solid preparations to increase the wettability of the product and therefore its disintegration); polymers (compounds that modify the time or the site of release of the active ingredient, for example in preparations with a prolonged release or gastro-resistant).

All of these agents listed before can be used for mono or multilayer tablets, swallowable, chewable, orosoluble or sublingual tablets or granules, for capsules or sachets.

With the term "excipient", it is intended here to refer to conventional excipient, i.e. inert compounds towards the active ingredient and the pharmaceutical form. Examples of different classes of these ingredients are: diluents (compounds added when the mass of the active ingredient is not sufficient for the preparation of the composition); lubricants (which avoid dust to adhere to the mechanical parts during the manufacturing process); aggregating (compounds which increase the cohesion of the powders); dyes (used to improve the presentation of certain pharmaceutical dosage forms, for example capsules, or to classify them according to the therapeutic category they belong to or to distinguish them from other similar products); sweeteners or flavoring (added to improve the organoleptic characteristics of the products); or antioxidants-antimicrobials (used to prolong the shelf life of the product.

Examples of such diluent agents, aggregating or binders, lubricants, glidants, disaggregating, solubilizers and/or pH regulators are the following: lightweight magnesium oxide, magnesium hydroxide, alginic acid, stearic acid, hydrogenated vegetable oils (palm, oleic or behenic), cocoa butter, cocoa mass, chitosan, yeast, sodium carboxymethylcellulose (CMC), pregelatinised maize starch, Pearlitol^{®} and Pearlitol^{®} Flash.

The most commonly used conventional excipients are the following: lactose, glucose, sucrose, mannite (or mannitol), kaolin, talc, bentonite, titanium dioxide, xylitol, maltitol, sorbitol, sucralose, acesulfame K, aspartame, neohesperidin, fructose, dextrose , maltose, "spraydried" malt, sodium aspartate, maltodextrin, sodium chloride, hydroxypropylmethylcellulose, erythritol, citrus extract, silica gel, vegetable fibers (such as pea fiber), flavorings and aromas, such as for example aroma of mint (peppermint, crispa, sweet), Badian anethole, vanilla, sage, liver, chicken, grapefruit, peach, orange, lemon or lime, sodium glutamate and fish flour.

According to a preferred aspect, the composition of the present invention is formulated as a orosoluble or orodispersible formulation (tablets or powder), i.e. quick release (fast) or immediate. For example in buccal formulations, PEA is preferably associated with mannitol, xylitol, magnesium stearate and silica gel, together with flavoring agents (e.g. orange flavor). In sublingual release formulations, PEA is associated with sucralose, Pearlitol^{®} Flash, magnesium stearate and silica gel, together with flavoring agents (e.g. peppermint flavor).

According to a further preferred embodiment of the invention, the composition is commercialized as intestinal release formulation (i.e. modified or enteric release). In such formulations, PEA is preferably associated with calcium phosphate, stearic acid, microcrystalline cellulose, magnesium stearate and silica gel.

According to a preferred aspect, PEA, which is used as raw material for the composition of the present invention, is substantially free of impurities (that is, it has a content of total impurities of 1.5% by weight or less) and is totally devoid of palmitoyl diethanolamide (PDEA) (that is, has a content of 0% in the PDEA). PDEA is the main product of degradation of PEA. Such impurities are meant as detected with gas chromatography (GC) or high-performance liquid chromatography (HPLC).

According to an even more preferred aspect, PEA powder that is used as raw material for the composition of the present invention has a particle size, such that only 0.2% of the particles have an average diameter of less than 100 µm. Such average particle diameter is intended to be determined by means of the calibrated sieves method (Official Pharmacopoeia (F.U.) of the Italian Republic).

It is a further object of the present invention the food supplement containing the composition of this invention in one of the previously described pharmaceutical forms.

With the term "food supplement" it is meant a food product intended to supplement the common diet and which is a concentrated source of nutrients, such as vitamins and minerals, or other substances with a nutritional or physiological effect, in unit dose form (see also Directive 2002/46 / EC of 10 June 2002 and Italian DLG n. 169 art. 2 of 21 May 2004).

Typically, the composition of the present invention is formulated and marketed in the form of tablet, capsule or powder/granules in sachet. Each tablet, capsule or sachet has a formulation according to the data of the tables below, which gives for each unit dose (tablet, capsule, sachet or other) the active ingredients, excipients and other components with the respective amounts in weight.

The following Table 2 shows some examples (2 Examples) of nutraceutical compositions according to the present invention. For each example, the active ingredients or plant extracts used in association with the PEA are reported, the respective amounts per unit dose, the dosage form used and the field of application of the supplement.

Preferred pharmaceutical forms of the invention generally contain a quantity of PEA (per unit dose) varying from 10 to 3,000 mg, more preferably from 50 to 1,500 mg, still more preferably from 150 to 1,000 mg.

**Table 2**

| **ACTIVE INGREDIENT/ PLANT EXTRACT** | **mg/unit dose** | **PHARMACEUTICAL FORM** | **USE IN THE FIELD** |
|---|---|---|---|
| | | | |

| **1** | | **Gastro-resistant tablet** | **Neurological** |
|---|---|---|---|
| PEA | 600 | | |
| Carnosic acid | 20 | | |
| Myrrh | 50 | | |
| Beta-caryophyllene | 10 | | |
| | | | |

| **2** | | **Three layers tablet** | **Neurological** |
|---|---|---|---|
| PEA | 300 | | |
| Carnosic acid | 10 | | |
| Myrrh | 25 | | |
| Beta-caryophyllene | 5 | | |
| | | | |
| | | | |
| | | | |
| | | | |
| | | | |
| | | | |

The daily dose of the product of the present invention generally varies from one unit to three-units doses.

The production process of the composition of the present invention is made using the technologies and well-known working conditions. In general, the process comprises mixing the active ingredients (or plant extracts containing them) together with the excipients and other components required in a mixer, in order to obtain the desired composition.

For example, the process of preparation of monolayer swallowable tablets, optionally also modified-release (prolonged gastro-resistant), or chewable tablets schematically comprises the following steps: weighing of the active components and excipients necessary to achieve the desired release, mixing all the components in a mixer (for example a rotating screw feeder), compressing the mixture obtained and subsequent painting with transparent, colored and/or gastro-resistant coating. In case of poorly flowing powders, before the compressing, a pre-compressing can be carried out with subsequent dry granulation, or, alternatively, a wet granulation can be effected.

For the multilayer (double / triple) tablets preparation process with a release fast / slow, the technique described in the patent EP 1225876 can be used with the following suggestions. The process schematically comprises the following steps: weighing the active ingredients and the excipients (specific for the desired release), divided into two/three different mixtures (according to the number of the layers); the separate mixing of each layer (for example in a rotating screw feeder), compressing the mixtures obtained for each layer (for example, using a PZ-TRE tabletting machine sold by B & D Italy), so as to obtain a multilayer tablet and the subsequent painting with transparent, colored and/or gastro-resistant coating. Also in this case, in the presence of poorly flowing powders, before compressing, a pre-compressing can be carried out with subsequent dry granulation, or, alternatively, a wet granulation can be executed.

The process for the preparation of orosoluble or sublingual tablets schematically comprises the same steps listed above, with the precaution that the production steps must be conducted in a temperature- and humidity-controlled environment (generally at a temperature around 25 °C and a humidity less than 30%) and that agents that induce a fast or immediate release of at least one of the active ingredients will have to be used. Even the orosoluble tablets can be prepared multilayered.

For the process of preparation of gastro-resistant granulates the techniques described in EP patent application 1861075 can be adopted. The granulates thus obtained may be packed in sachets or jars.

The process for the preparation of effervescent granules schematically comprises the following steps: weighing the active components and the excipients, mixing all the components in a mixer (for example a rotating screw feeder) and granulating the obtained mixture. In case of poorly flowing powders, before the granulation, a pre-compressing can be carried out with subsequent dry granulation, or, alternatively, a wet granulation can be effected.

In the case of effervescent granules, the production steps must be conducted in an environment with controlled temperature and humidity (generally at a temperature of 25 ° C and a humidity of less than 30%).

The method for the preparation of hard gelatine or HPMC capsules filled with liquids schematically comprises the following steps: weighing the ingredients and the excipients both liquid and solid, mixing all components thus creating an emulsion with a mixer for liquids and finally encapsulating the obtained emulsion with an appropriate machine (for example the Model Liqfil S.80 of Shionogi).

Also the capsules so obtained may undergo subsequent coating with transparent, colored and/or gastro-resistant coating.

Some (non-limiting) examples of the present invention are given below.

### DESCRIPTION OF THE FIGURE

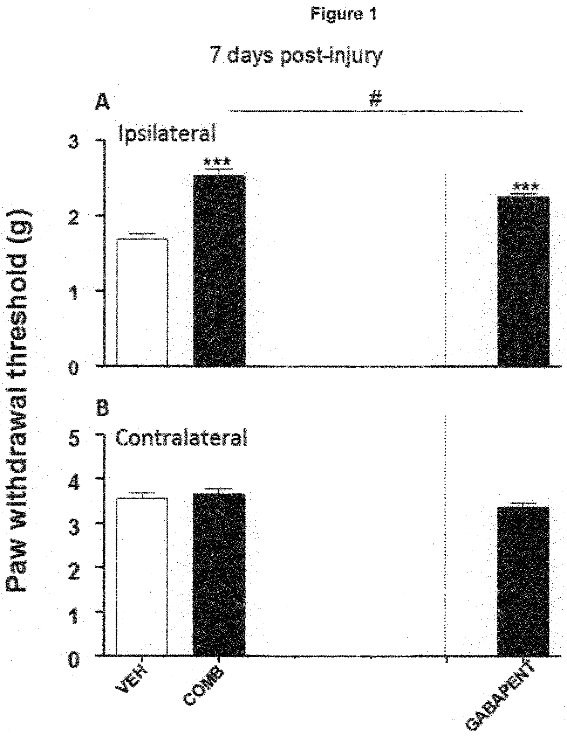

**Figure 1**: it shows the effects of the vehicle (VEH), the nutraceutical composition of the invention (COMB) and gabapentin (GABAPENT) on tactile allodynia measure with the Von Frey test after 7 days from sciatic nerve damage in: A) Ipsilateral paw; B) or in the contralateral control paw . Data represent the MEAN ± SEM values. Difference from the control group (VEH): ***p<0.0001. Difference between GABAPENT and COMB: # p<0.05

### EXAMPLES

### Example 1 - Monolayer gastro-resistant tablets containing PEA in association with carnosic acid, myrrh and beta-caryophyllene

The following amounts of components were weighed separately. Firstly PEA (600 mg) and stearic acid (80 mg) were used to prepare a retard granulate according to the following steps: melting stearic acid (to a temperature of about 80 ° C); covering PEA with molten stearic acid; cooling, granulating and calibrating the granulate. Subsequently the "retard" granulate previously obtained, 20 mg of carnosic acid (from a *Rosmarinus officinalis* extract containing 65% by weight of carnosic acid), 50 mg of myrrh (a *Commiphora myrrha* extract), 100 mg beta-caryophyllene (from a *Piper Nigrum* extract containing 80% by weight of beta-caryophyllene), 150 mg of calcium phosphate, 210 mg of microcrystalline cellulose, 5 mg of magnesium stearate and 5 mg of gel silica (intended as amounts per tablet unit dose) were mixed in a rotating screw feeder.

The mixture thus obtained was introduced into a tabletting machine, so as to obtain oblong tablets of about 1.2 g.

The tablets thus obtained were then subjected to painting with transparent coating.

### Example 2 - Three layers tablets containing PEA in association with carnosic acid, myrrh and beta-caryophyllene

The following amounts of components (intended as amounts per tablet unit dose) were weighed separately. Firstly PEA (300 mg) and stearic acid (40 mg) were used to prepare a "retard" granulate according to the following steps: melting stearic acid (to a temperature of about 80 ° C); covering PEA with molten stearic acid; cooling, granulating and calibrating the granulate. The resulting "retard" granulate is then divided into two aliquots having the same weight.

Subsequently 10 mg of carnosic acid (from a *Rosmarinus officinalis* extract containing 65% by weight of carnosic acid), 25 mg of myrrh (a *Commiphora myrrha* extract), 50 mg beta-caryophyllene (from a *Piper Nigrum* extract containing 80% by weight of beta-caryophyllene), 75 mg of calcium phosphate, 105 mg of microcrystalline cellulose, 2.5 mg of magnesium stearate and 2.5 mg of gel silica (intended as amounts per tablet unit dose) were mixed in a rotating screw feeder. The resulting mixture is set aside for subsequent processing.

A three layers tablet is then prepared by compressing in a PZ-TRE tabletting machine (sold by B & D Italy) the following layers: the first layer is composed of one of the two aliquots of the "retard" granulate containing PEA, the second layer is composed of the mixture containing carnosic acid, myrrh and beta-caryophyllene and the third layer is composed of the second aliquot the "retard" granulate containing PEA.

### Example 3 - Effect of a nutraceutical preparation on neuropathic pain in mice

Neuropathic pain (NeP) is a chronic pain "arising as a direct consequence of a lesion or disease affecting the somatosensory system" (definition according to the International Association for the Study of Pain - IASP).

The effect of a nutraceutical composition containing palmitoylethanolamide (PEA), mirrh, carnosic acid and beta-cariophyllene in an animal model of neuropathic pain (the chronic constriction injury - CCI) of the sciatic nerve is here reported.

In particular, the composition reported in Example 4 was tested in this experiment, while gapapentin, an approved medication for neuropathic pain treatment, was used as a positive comparator.

The tablets prepared according to Example 2 were grinded using an electric grinder and the resulting powder was suspended in distilled water with the addition of 5% (final volume) of Tween^{®} 80 to facilitate PEA dissolution. The final concentration per unit dose was: 150 mg of PEA, 2.5 of beta-cariophyllene, 5 mg of carnosic acid and 12,5 mg of mirrah /kg/10 ml.

The 100 mg capsules of gabapentin (sold by TEVA PHARMA) were grinded and suspended in distilled water to obtain a solution. The final concentration per unit dose was 50 mg/kg/10ml.

The above procedures applied to both tablets of the invention and gabapentin capsules were carried out immediately before the oral administration to the animals.

The study was conducted in accordance with the European Community Council Directive for Care and Use of Laboratory Animals and the National Institutes of Health Guidelines for Care and Use of Laboratory Animals.

Male mice (CDI from Charles River), 5 weeks old, weighing about 25 grams were housed in groups of 5 in a room with artificial 12:12 hours light/dark cycle (light on at 7:00 a.m.), constant temperature (20-22°C) and humidity (45-55%). All animals were handled once daily for 5 minutes for one week before the beginning of the experiments. During the experiments, mice were offered free access to tap water and food pellets (4RF18, Mucedola, Settimo Milanese, Italy).

The test compounds were administered orally twice a day (at 9.00 a.m. and at 5.00 p.m.) for 7 consecutive days. Tests were performed starting 30 minutes after the morning injection.

For the induction of neuropathic pain, the chronic constriction injury (CCI) model was used.

Mice were anesthetized by inhalation of a mixture of isoflurane and oxygen. The anesthetic was delivered by a facemask connected with a gas anesthesia machine equipped with an oxygen source and a precision vaporizer. Isoflurane was used in a concentration of 4-5% for anesthesia induction and of 1-2% for maintenance. Each mouse was constantly monitored to avoid excess cardiac and respiratory depression and insufficient anesthesia.

To perform the CCI, the left sciatic nerve of the animal was exposed at the level of the mid-thigh. Three ligatures were then loosely tied around the nerve, using a non absorbable surgical suture thread.

Behavioral tests were performed 7 days after nerve injury.

Changes in nociceptive thresholds were evaluated using an Electronic von Frey device (Ugo Basile) at 7 days post injury.

Immediately after the morning drug administration, mice were placed in individual plastic boxes with a metal mesh floor and allowed to adapt for 30 min. After the adaptation period, the test began.

Gradually increased strength was applied to filaments through the mesh floor perpendicularly to the plantar surface of the hind paw. The intensity of mechanical stimuli was increased (range from 0.1 to 5 g) until the hind paw was withdrawn. Threshold pressure values were recorded. The nociceptive threshold for each paw was obtained in triplicate and the mean of the three measurements was calculated.

Animals were observed for any clinical signs that could be specific to treatments and were weighed and examined for health status.

Withdrawal thresholds of the ipsilateral paw obtained during the tests were analyzed using a one ways ANOVA with "treatment" as between factor. Time points (day 7) were analyzed separately. Newmann-Keuls was used as the post-hoc. To allow comparisons between treatments, the effect of nutraceutical composition of the invention and gabapentin were analyzed together. As a control also withdrawal thresholds data of the contralateral paw was recorded and analyzed.

Results: Nutraceutical composition of the invention reduced tactile allodynia in neuropathic mice as assessed by the Von Frey test

At day 7, using the t-test, ipsilateral vs contralater paw response in the vehicle treated group was analyzed. Results showed a significant (t=3.3; df=14 p<0.0001) lower withdrawal threshold for the ipsilateral paw (ligated) compared to the contralateral (sham operated) indicating a significant development of allodynia following neuronal injury. Subsequently a one-way ANOVA was used to evaluate the effect of treatment revealing a significant overall effect of treatment [F(4,37)=21.27, p<0.001]. Post hoc tests showed that, compared to vehicle, both treatments elicited a highly significant attenuation of neuropathic pain (p<0.001). Notably, the administration of the nutraceutical composition resulted even more efficacious than gabapentin (p<0.05).

At day 7 ANOVA revealed no drug effects on withdrawal thresholds of the contralateral paw thus showing high specificity of action.

Results showed the nutraceutical preparation containing 300 mg of PEA, 5 mg of betacariophyllene, 10 mg carnosic acid and 25 mg of mirrah significantly attenuated neuropathic pain following sciatic nerve ligation.

Such results are also shown in Figure 1.

## Claims

1. Food and/or nutraceutical composition comprising palmitoylethanolamide (PEA) in association with a plant extract from *Commiphora myrrha (Nees) Engl.,* beta-caryophyllene and carnosic acid.

2. The composition according to claims 1, comprising a further active ingredient, which is selected from the group comprising: S-Adenosyl-L-methionine (SAMe), 5-HTP (5-hydroxytryptophan), acetyl I-carnitine, agmatine, alkylamides, asperuloside, astaxanthin, baicalin, beta-sitosterol, boswellic acids, bromeline, carnosol, catechins, choline, chondroitin sulphate, citicoline, coenzyme Q10, cumarin , curcumin, D-mannose, echinacoside, ellagic acid, epicatechins , epigallocatechin gallate , eugenol, flavonoids, forskolin, GABA (gamma-aminobutyric acid), galactose , alfa- and gamma-mangosteen, ginesteine, glucosamine, GPC (glycerophosphorylcholine), GPE (glycerophosphoethanolamide), HMB (Hydroxymethylbutyrate), homotaurine/tramiprosate, hyaluronic acid, hydroxytyrosol , inositol, iridoid glycosides, L-Glutathione, loperamide, L-Theanine, lutein, lycopene, melatonin, N-acetylcistein, NADH (nicotinamide adenine dinucleotide reduced), oleuropein, PABA (Para-aminobenzoic acid), phenolic acids, phosphatidylcholine, phosphatidylserine, phosphoserine, policosanols, polyphenols, proanthocyanidins, quercetin, resveratrol, rosmarinic-acid, rutamarin, salicin, salvinorin-A, Serratiopeptidasi, SOD (superoxide dismutase), squalene, sulforaphane, tetrahydrocurcuminoids, teupolioside, trans-resveratrol, troxerutin, tyrosol, xanthones and zeaxanthin.

3. The composition according to claim 1, further comprising a release modifying agent, which is selected from the group comprising the following classes: diluents, aggregating agents or binders, lubricants, glidants, disintegrants, solubilizing agents and pH regulators.

4. The composition according to claim 3, wherein the release modifying agent is selected from the group comprising: magnesium oxide light, magnesium hydroxide, alginic acid, stearic acid, hydrogenated vegetable oils from palm, oleic, behenic oil, cocoa butter, cocoa mass, chitosan, yeast, carboxymethylcellulose (CMC) sodium, pregelatinised maize starch, di- tri-carboxylic acids, bi-hydrated and mono hydrate sodium citrate, sodium carbonate, sodium bicarbonate, potassium bicarbonate, citric acid, tartaric acid, adipic acid, monosodium phosphate and magnesium hydroxycarbonate.

5. Food supplement comprising the composition according to claim 1 in a pre-dosed pharmaceutical form, which is in a modified-release form.

6. The composition according to claim 1, in the pharmaceutical form of buccal, gastro-resistant, multilayered, swallowable, chewable, gastro-filmed or sublingual tablet, or hard capsule filled with liquid and swallowable, chewable, or granulate.

7. Process for the preparation of the composition according to claim 1, comprising weighing the individual components, blending the same and transferring the obtained mixture in a machine able to confer the final pharmaceutical form.

8. The composition according to anyone of claims from 1 to 6, for use as a coadjuvant agent to supplement common diet food with the active ingredients contained therein, in the treatment of neuropathic pain.

## Patentansprüche

1. Lebensmittel- und/oder nutraceutische Zusammensetzung, umfassend Palmitoylethanolamid (PEA) in Verbindung mit einem Pflanzenextrakt aus *Commiphora myrrha* (Nees) Engl., Beta-Caryophyllen und Carnosinsäure.

2. Zusammensetzung nach Anspruch 1, umfassend einen weiteren Wirkstoff, der ausgewählt ist aus der Gruppe umfassend: S-Adenosyl-L-methionin (SAMe), 5-HTP (5-Hydroxytryptophan), Acetyl-L-Carnitin, Agmatin, Alkylamide, Asperulosid, Astaxanthin, Baicalin, Beta-Sitosterol, Boswelliasäuren, Bromelain, Carnosol, Catechine, Cholin, Chondroitinsulfat, Citicolin, Coenzym Q10, Cumarin, Curcumin, D-Mannose, Echinacosid, Ellagsäure, Epicatechine, Epigallocatechingallat, Eugenol, Flavonoide, Forskolin, GABA (Gamma-Aminobuttersäure), Galactose, Alpha- und Gamma-Mangostin, Genistein, Glucosamin, GPC (Glycerophosphorylcholin), GPE (Glycerophosphoethanolamid), HMB (Hydroxymethylbutyrat), Homotaurin/Tramiprosat, Hyaluronsäure, Hydroxytyrosol, Inositol, Iridoidglykoside, L-Glutathion, Loperamid, L-Theanin, Lutein, Lycopin, Melatonin, N-Acetylcystein, NADH (reduziertes Nicotinamidadenindinukleotid), Oleuropein, PABA (Para-Aminobenzoesäure), Phenolsäuren, Phosphatidylcholin, Phosphatidylserin, Phosphoserin, Policosanole, Polyphenole, Proanthocyanidine, Quercetin, Resveratrol, Rosmarinsäure, Rutamarin, Salicin, Salvinorin-A, Serratiopeptidase, SOD (Superoxiddismutase), Squalen, Sulforaphan, Tetrahydrocurcuminoide, Teupoliosid, Trans-Resveratrol, Troxerutin, Tyrosol, Xanthone und Zeaxanthin.

3. Zusammensetzung nach Anspruch 1, ferner umfassend ein freisetzungsmodifizierendes Mittel, das ausgewählt ist aus der Gruppe umfassend die folgenden Klassen: Verdünnungsmittel, Aggregationsmittel oder Bindemittel, Schmiermittel, Fließregulierungsmittel, Sprengmittel, Solubilisierungsmittel und pH-Regulatoren.

4. Zusammensetzung nach Anspruch 3, wobei das freisetzungsmodifizierende Mittel ausgewählt ist aus der Gruppe umfassend: leichtes Magnesiumoxid, Magnesiumhydroxid, Alginsäure, Stearinsäure, hydrierte pflanzliche Öle aus Palmöl, Ölsäureöl, Behenöl, Kakaobutter, Kakaomasse, Chitosan, Hefe, Natriumcarboxymethylcellulose (CMC), vorverkleisterte Maisstärke, Di- und Tricarbonsäuren, Dinatriumcitrat-Dihydrat und Natriumcitrat-Monohydrat, Natriumcarbonat, Natriumbicarbonat, Kaliumbicarbonat, Citronensäure, Weinsäure, Adipinsäure, Mononatriumphosphat und Magnesiumhydroxycarbonat.

5. Nahrungsergänzungsmittel, umfassend die Zusammensetzung nach Anspruch 1 in einer vordosierten pharmazeutischen Form, die eine Form mit modifizierter Freisetzung ist.

6. Zusammensetzung nach Anspruch 1 in der pharmazeutischen Form einer bukkalen, magensaftresistenten, mehrschichtigen, schluckbaren, kaubaren, magensaftbefilmten oder sublingualen Tablette oder einer mit Flüssigkeit gefüllten und schluckbaren oder kaubaren Hartkapsel oder eines Granulats.

7. Verfahren zur Herstellung der Zusammensetzung nach Anspruch 1, umfassend das Abwiegen der einzelnen Komponenten, das Vermischen derselben und das Überführen der erhaltenen Mischung in eine Maschine, die geeignet ist, die endgültige pharmazeutische Form zu verleihen.

8. Zusammensetzung nach einem der Ansprüche 1 bis 6 zur Verwendung als adjuvantes Mittel zur Ergänzung der üblichen Ernährung mit den darin enthaltenen Wirkstoffen bei der Behandlung neuropathischer Schmerzen.

## Revendications

1. Composition alimentaire et/ou nutraceutique comprenant de la palmitoyléthanolamide (PEA) en association avec un extrait végétal de *Commiphora myrrha* (Nees) Engl., du bêta-caryophyllène et de l'acide carnosique.

2. La composition selon la revendication 1, comprenant un ingrédient actif supplémentaire, qui est choisi dans le groupe comprenant: la S-adénosyl-L-méthionine (SAMe), le 5-HTP (5-hydroxytryptophane), l'acétyl-L-carnitine, l'agmatine, les alkylamides, l'asperuloside, l'astaxanthine, la baicaline, le bêta-sitostérol, les acides boswelliques, la bromélaïne, le carnosol, les catéchines, la choline, le sulfate de chondroïtine, la citicoline, la coenzyme Q10, la coumarine, la curcumine, le D-mannose, l'échinacoside, l'acide ellagique, les épicatéchines, le gallate d'épigallocatéchine, l'eugénol, les flavonoïdes, la forskoline, le GABA (acide gamma-aminobutyrique), le galactose, l'alpha- et le gamma-mangoustan, les génistéines, la glucosamine, la GPC (glycérophosphorylcholine), le GPE (glycérophosphoéthanolamide), le HMB (hydroxyméthylbutyrate), l'homotaurine/tramiprosate, l'acide hyaluronique, l'hydroxytyrosol, l'inositol, les glycosides iridoïdes, le L-glutathion, le lopéramide, la L-théanine, la lutéine, le lycopène, la mélatonine, la N-acétylcystéine, le NADH (nicotinamide adénine dinucléotide réduit), l'oleuropéine, le PABA (acide para-aminobenzoïque), les acides phénoliques, la phosphatidylcholine, la phosphatidylsérine, la phosphosérine, les policosanols, les polyphénols, les proanthocyanidines, la quercétine, le resvératrol, l'acide rosmarinique, la rutamarine, la salicine, la salvinorine A, la serratiopeptidase, la SOD (superoxyde dismutase), le squalène, le sulforaphane, les tétrahydrocurcuminoïdes, le teupolioside, le trans-resvératrol, la troxérutine, le tyrosol, les xanthones et la zéaxanthine.

3. La composition selon la revendication 1, comprenant en outre un agent modificateur de libération, qui est choisi dans le groupe comprenant les classes suivantes : diluants, agents d'agrégation ou liants, lubrifiants, agents d'écoulement, désintégrants, agents solubilisants et régulateurs de pH.

4. La composition selon la revendication 3, dans laquelle l'agent modificateur de libération est choisi dans le groupe comprenant : l'oxyde de magnésium léger, l'hydroxyde de magnésium, l'acide alginique, l'acide stéarique, les huiles végétales hydrogénées de palme, l'huile oléique, l'huile béhénique, le beurre de cacao, la pâte de cacao, le chitosane, la levure, la carboxyméthylcellulose sodique (CMC), l'amidon de maïs prégélatinisé, les acides dicarboxyliques et tricarboxyliques, le citrate de sodium dihydraté et monohydraté, le carbonate de sodium, le bicarbonate de sodium, le bicarbonate de potassium, l'acide citrique, l'acide tartrique, l'acide adipique, le phosphate monosodique et l'hydroxycarbonate de magnésium.

5. Complément alimentaire comprenant la composition selon la revendication 1 sous une forme pharmaceutique prédosée, qui est sous une forme à libération modifiée.

6. La composition selon la revendication 1, sous la forme pharmaceutique d'un comprimé buccal, gastro-résistant, multicouche, à avaler, à mâcher, gastro-pelliculé ou sublingual, ou d'une gélule dure remplie de liquide et destinée à être avalée, mâchée, ou sous forme de granulé.

7. Procédé pour la préparation de la composition selon la revendication 1, comprenant la pesée des composants individuels, leur mélange, puis le transfert du mélange obtenu dans une machine apte à conférer la forme pharmaceutique finale.

8. La composition selon l'une quelconque des revendications 1 à 6, destinée à être utilisée comme agent adjuvant pour compléter l'alimentation courante par les ingrédients actifs qu'elle contient, dans le traitement de la douleur neuropathique.
